# EUROPEAN PATENT APPLICATION

(11) **EP 0 761 687 A1**
(43) Date of publication of application: **12.03.1997**
(21) Application number: 95918759.2
(22) Date of filing: 22.05.1995
(51) Int. Cl.: C07K 16/00

(54) **PROCESS FOR PREPARING ANTIBODY**

(30) Priority: 24.05.1994 JP 109329/94
(71) Applicant: SAGAMI CHEMICAL RESEARCH CENTER, Sagamihara-shi, Kanagawa 229 (JP)
(72) Inventor: KATO, Seishi, Kanagawa 228 (JP); SUGANO, Sumio, Tokyo 167 (JP)
(74) Representative: VOSSIUS & PARTNER
(86) International application number: JP9500973
(87) International publication number: WO9532224

(57) **Abstract**

A process for preparing an antibody against a protein, which comprises joining a DNA coding for the protein with an expression vector containing a promoter originating in animal cells and injecting the resultant vector into an animal body. This process facilitates the preparation of the antibody useful as a medicine, diagnostic agent or research reagent.

## Description

### Technical Field

The present invention relates to a process for preparing an antibody without purification of a protein antigen. This process facilitates the preparation of antibodies useful as a medicine, diagnostic agent or research reagent.

### Background

An antibody has widely been used as a research reagent for detection, purification, removal, or inhibiting activity of a particular protein, since it can recognize the protein and bind to it. Recently the antibody has been applied to the use as a medicine or diagnostic agent as well as a research reagent. In the conventional method for preparing the antibody, animals such as rabbit or mouse were injected with a purified protein antigen, followed by isolating the antibody produced in serum. The mass production of purified protein antigen required much time and labor. Thus more simple process for preparing the antibody has been desired.

Recently it has been reported that intramuscular injection with an expression vector DNA carrying a gene encoding an influenza virus envelope protein to mouse resulted in the production of the viral envelope protein in the mouse body and the production of the antibody against the protein [Ulmer et al., Science 259:1745-1749, 1993; Ginsbert et al., "Vaccines 93"]. As a result the mouse acquired the immunity against the virus so that this expression vector has attracted attention as a new type of vaccine, called a DNA vaccine.

The promoters of the expression vectors used in the above reports were derived from viruses such as Rous sarcoma virus or cytomegalovirus, and the expressed genes encoded the viral envelope proteins which were released out of the cells. It is unknown whether such antibody production is observed when a non-viral promoters are used or when the expression product is a viral intracellular protein or when a non-viral protein is expressed. In general an antibody production occurs after an antigen presentation by macrophage which uptakes an antigen from an extracellular fluid such as blood or lymph. Thus it is considered that the antibody production against non-secretory proteins hardly occur.

There is a report that antibodies against secretory proteins such as human growth hormone or human alphalantitrypsin were produced in mouse blood when expression vectors carrying genes of these proteins downstream of promoters such as human beta-actin promoter or cytomegalovirus promoter were constructed and gold particles coated with these plasmid were introduced into mouse skin cells using a microprojectile [Tang et al., Nature 356:152-154,1992]. The report, however, describes that the injection of the plasmids does not cause such antibody production. Furthermore there is no such trial for non-secretory proteins in this paper.

### Disclosure of Invention

The inventors completed the present invention by discovering that an antibody against a viral protein or a human protein expressed in the cell is produced in the serum of the animal injected with an expression vector which carries a promoter functioning in the animal cell and a DNA encoding the target protein.

The present invention provides a process for preparing an antibody against a protein, which comprises subcloning a DNA encoding the target protein into an expression vector carrying a promoter derived from an animal cell and then injecting the obtained expression vector into an animal.

Furthermore the present invention provides a process for preparing an antibody against a non-viral protein, which comprises subcloning a DNA encoding the target protein into an expression vector carrying a promoter functioning in an animal cell and then injecting the obtained expression vector into an animal.

The protein of the present invention includes any proteins encoded by genes of organisms such as viruses, bacteria, plants, or animals and the like. These proteins can be categorized into secretory proteins and non-secretory proteins. Furthermore non-secretory proteins can be categorized into membrane proteins, cytoplasmic proteins, nuclear proteins, and organelle proteins etc. by their location in the cell. Secretory proteins include components of extracellular fluids such as blood or lymph, cytokines, extracellular matrices and so on. Membrane proteins include receptors, transporters, ion channels and so on. Cytoplasmic proteins include cytoskeletons, metabolic enzymes, signal transducing factors, protein-synthesis-related proteins (e.g. ribosomal proteins), protein-degradation-related proteins (e.g. proteasome components) and the like. Nuclear proteins include nuclear skeletons, transcription factors, replication factors and so on. Organelle proteins include various proteins (electron-transporting system, degrading enzymes etc.) which are components of mitochondria, lysosome, peroxisome, microsome and so on. Furthermore the present invention includes artificial proteins encoded by synthetic gene designed de novo.

The DNA encoding the target protein includes any DNA such as genome DNA, cDNA, or synthetic DNA as long as the DNA contains the open reading frame starting form an initiation codon to a stop codon.

The promoter functioning in the animal cell includes any promoter derived from a virus or an animal cell. As representative examples of the promoters, there may be mentioned: virus-derived promoters such as an early or late promoter of SV40, a late promoter of adenovirus, LTR of retrovirus, a promoter of Rous sarcoma virus, a promoter of cytomegalovirus, etc.; animal cell-derived promoters such as a promoter of thymidine kinase, a promoter of metallothionein, a promoter of beta-actin, a promoter of elongation factor-1alpha, a promoter of ribosomal protein etc.

The DNA encoding the target protein can be subcloned into the expression vector carrying the above promoter using known techniques in genetic engineering.

The expression vector carrying the target gene can be any form such as circular double-stranded DNA, circular single-stranded DNA, linear double-stranded DNA, phage particle containing these DNAs. The expression vector DNAs may be injected in any form such as DNA dissolved or suspended in saline or buffer solution, or DNA encapsuled in liposome.

The animal used for the injection include mammals such as mouse, rat, rabbit etc., or birds such as chicken etc. which have generally been used for preparing an antibody. The injection of a DNA sample into the animal body may be carried out intra-muscularly, intra-abdominally, or intravenously using a syringe. The amount of DNA to be injected depends on the animal used, and generally is used in the range of 0.1 ng to 1 g per body. The injection of the DNA sample may be carried out once or preferably more than twice at the appropriate intervals to succeed the antibody production.

The antibody production is detected by examining the binding activity of the serum isolated from blood with the antigen protein. For example, known methods such as immuno-precipitation, enzyme-immunoassay, antibody staining etc. can be used. After confirming the antibody production in the serum using these methods, the serum itself or the antibody purified by affinity column chromatography can be used as a polyclonal antibody sample. According to the conventional method, monoclonal antibody can also be prepared using the spleen isolated from the animal which acquired immunity.

### Brief Description of the Drawings

Figure 1 shows the structure of pEF204-T which is the SV40 T antigen expression vector carrying a promoter of human elongation factor-1alpha.

Figure 2 shows the structure of pEF321-CD44 which is the human CD44 antigen expression vector carrying a promoter of human elongation factor-1alpha.

### Best Mode for Carrying Out the Invention

The present invention will now be described by way of examples, but various changes and modifications can be made without departing from the invention.

### EXAMPLE 1 Preparation of antibody against SV40 T antigen

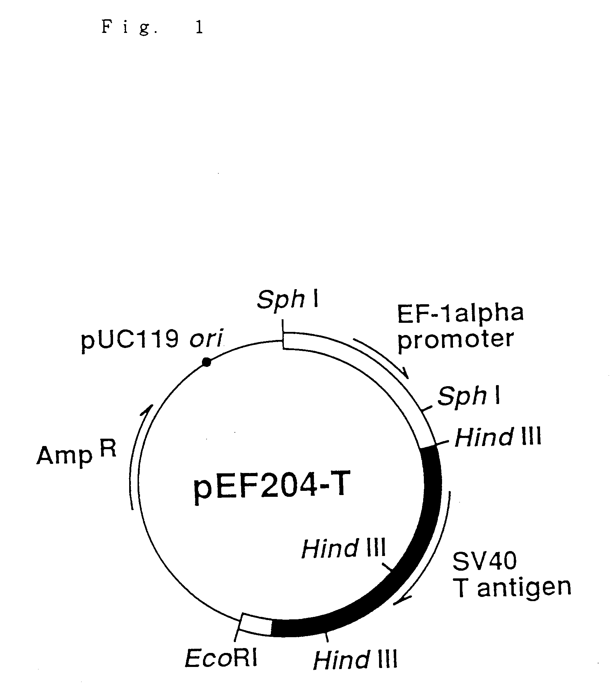

The expression vector pEF204-T which contains the coding region of SV40 T antigen located downstream of the promoter of human elongation factor-1alpha (Fig.1, Kim et al., Gene 91:217-223,1990) was employed. The expression vector pEF204-T was amplified in E. coli cells and purified by equilibrium centrifugation in CsCl density gradient. The obtained plasmid was dissolved in phosphate buffer (PBS: 145 mM NaCl, 2.68 mM KCl, 8.09 mM Na₂HPO₄, 2 mM KH₂PO₄, pH 7.2) so as to render the concentration to be 2 µg/µl.

Six female BALB/C mice (6-8 weeks old) were used. Before injection, control pre-immune blood of about 200 µl was sampled from the eye of each mouse using a capillary glass. Then 25 µl each of the PBS solution of plasmid prepared above, i.e., total 50 µl per mouse, was injected into musculus quadriceps of both right and left legs of three mice using syringe of 26 gage. The remaining three mice were injected intra-abdominally with 50 µl of the PBS solution of plasmid. These experiments were performed in the animal breeding facilities of P2 level.

After three weeks about 200 µl of blood sample was taken from the eye, and then the PBS solution of plasmid was injected again in the same manner as the first injection. The last blood sampling was performed after further three weeks. The collected blood was stored at 4 °C in a refrigerator overnight to clot the blood, and then the supernatant was collected by centrifugation at 8000 g for 5 min. This supernatant was added with NaN₃ so as to make the concentration to be 0.01 % and then stored at 4 °C.

The monkey kidney cell line COS7 cells expressing SV40 T antigen were cultured on the cover glass. When the cells contacted each other, the cover glass was immersed for 15 minutes in the ethanol-acetone (1:1) solution cooled at -20 °C to fix the cells, dried at room temperature, and immersed in PBS. The serum isolated from the mouse blood was diluted with PBS containing 0.5 % bovine serum albumin and placed onto the cells on the cover glass pulled out from PBS. The cover glass was left for one hour in the tapper to prevent drying up the cells and thoroughly washed with PBS to remove the serum. Then the FITS-labeled anti-mouse IgG antibody (purchased from DAKO) diluted 200-fold with PBS containing 0.5 % bovine serum albumin was placed on the cells and left for one hour at room temperature. After thorough washing with PBS, the fluorescence from the nucleus in which the SV40 T antigen is specifically expressed was observed using a fluorescent microscopy. As shown in Table 1, all sera collected from the mice which were intra-muscularly injected once contained the antibody against SV40 T antigen. Two shots of administration caused around 10-fold increase of the antibody titer. On the other hand, only one mouse produced the antigen in the case of intra-abdominal injection.

**Table 1**

| The results of antibody production examination | | | |
|---|---|---|---|
| Mouse | Pre-immune serum | after 3 weeks | after 6 weeks |
| | | | |

| Intramuscular injection | | | |
|---|---|---|---|
| A | - | ++ | ++++ |
| B | - | + | +++ |
| C | - | ++ | ++++ |

| Intra-abdominal injection | | | |
|---|---|---|---|
| A | - | - | - |
| B | - | - | - |
| C | - | + | ++ |
| +: nucleus was specifically stained with 10-fold dilution ++: nucleus was specifically stained with 30-fold dilution +++: nucleus was specifically stained with 90-fold dilution ++++: nucleus was specifically stained with 270-fold dilution | | | |

### EXAMPLE 2 Preparation of antibody against human CD44 antigen

The clone pKA1-CD44 containing full-length cDNA of human CD44 antigen was obtained by determining the nucleotide sequence of the clones randomly selected from the human lymphoma cell line U937 cDNA library (described in EP-426455-A). The determination of the entire nucleotide sequence of a cDNA insert of about 1.4 kbp showed that the obtained nucleotide sequence agreed with that described in the literature (Screaton et al., Proc.Natl.Acad.Sci.USA 89:12160-12164,1992). It has been known that CD44 forms various molecular species by alternative splicing. The obtained clone encoded the hematopoietic cell type CD44.

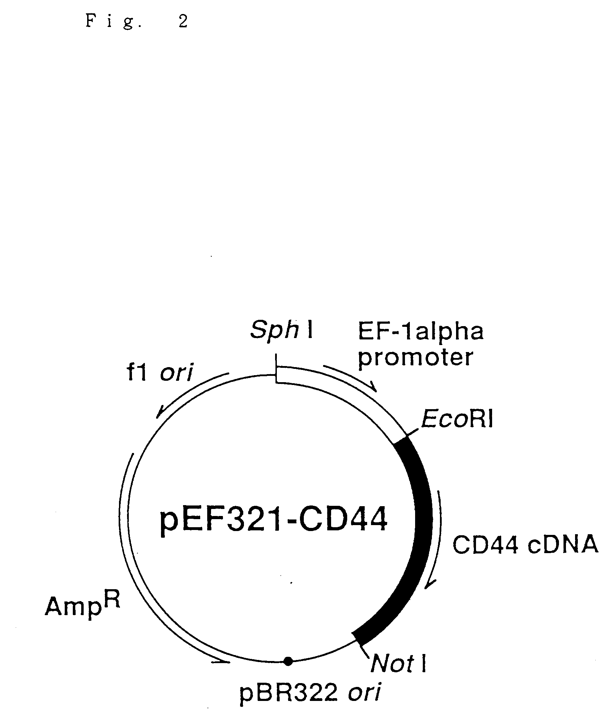

pEF321-T (Kim et al., Gene 91:217-223,1990) was digested with SphI and EcoRI. After agarose gel electrophoresis, the fragment of about 1.2 kbp containing a promoter of human elongation factor-1alpha was isolated from the gel. After pKA1-CD44 was digested with SphI and EcoRI, the longer fragment containing cDNA was isolated from the gel in the same manner. Both fragments were ligated to construct pEF321-CD44 (Fig. 2) which is an expression vector of human CD44 antigen in a mammalian cell.

The PBS solution containing 100 µg of human CD44 antigen expression vector pEF321-CD44 was injected intra-muscularly into mice under the same condition as described in EXAMPLE 1. The injection was carried out twice at an interval of 3 weeks and then blood was taken to prepare the serum.

The pKA1-CD44 was used as a template for in vitro translation in the rabbit reticulocyte lysate (Promega's T_{N}T in vitro transcription/translation kit). In this reaction [³⁵S]methionine (Amersham) was added to label the translation product with radioisotope. The reaction was done under the condition described in the protocol attached to the kit. Four µl of the reaction mixture containing in vitro translation product was mixed with 20 µl of the serum prepared above and 200 µl of the NET gel buffer (50 mM Tris-HCl, pH 7.5, 150 mM NaCl, 0.1 % NP-40, 1 mM EDTA, 0.25 % gelatin, 0.02 % NaN₃) and left for 1 hour on the ice. Into this solution 10 mg of protein A-bound Sepharose CL-4B (Pharmacia) was added and the whole was left for 1 hour at 4 °C. After centrifugation, the gel was washed twice with 1 ml of the NET gel buffer and then twice with 1 ml of 10 mM Tris buffer (pH 7.5). The gel was suspended in 20 µl of the sampling buffer (50 mM Tris-HCl, pH 6.8, 100 mM DTT, 2 % SDS, 0.1 % bromophenol blue, 10 % glycerol) and heated for 5 minutes at 100 °C. After centrifugation the supernatant was applied on SDS-polyacrylamide gel electrophoresis and the immuno-precipitated product was analyzed by autoradiography. As a result, only sample mixed with the serum of plasmid-injected mouse showed the band of molecular mass of about 46 kDa which correspond to that of CD44, thereby the production of the antibody was confirmed.

### INDUSTRIAL AVAILABILITY

The present invention provides a simple process for preparing an antibody against a target protein without purification of the protein antigen which requires complicated procedures and much time. The process of the present invention enables to prepare an antibody against the target protein if the DNA encoding the protein is available. The obtained antibodies are useful as medicines, diagnostic agents or research reagents.

## Claims

1. A process for preparing an antibody against a protein, which comprises subcloning a DNA encoding the protein into an expression vector carrying a promoter derived from an animal cell and then injecting the obtained expression vector into an animal.

2. A process for preparing an antibody against a non-viral protein, which comprises subcloning a DNA encoding the protein into an expression vector carrying a promoter functioning in an animal cell and then injecting the obtained expression vector into an animal.

3. The process of claim 2 wherein the promoter functioning in an animal cell is a promoter derived from an animal cell.

4. The process of any one of claims 1 to 3 wherein the protein is a non-secretory protein.

5. The process of any one of claims 1 to 4 wherein the promoter is a promoter of human elongation factor-1alpha.

6. The process of any one of claims 1 to 5 wherein the DNA encoding the protein is a human cDNA.
